(19) **Europäisches Patentamt / European Patent Office / Office européen des brevets**

(11) **EP 1 641 443 B1**

(12) # EUROPEAN PATENT SPECIFICATION

(45) Date of publication and mention
of the grant of the patent:
**14.01.2009 Bulletin 2009/03**

(51) Int Cl.:
*A61K 31/10* (2006.01)     *A61K 45/06* (2006.01)
*A61K 31/125* (2006.01)     *A61Q 17/04* (2006.01)
*A61P 17/16* (2006.01)

(21) Application number: **03733017.2**

(22) Date of filing: **30.05.2003**

(86) International application number:
**PCT/IT2003/000340**

(87) International publication number:
**WO 2004/105741 (09.12.2004 Gazette 2004/50)**

(54) **COSMETIC AND/OR PHARMACEUTICAL COMPOSITION COMPRISING DIMTHYLSULPHONE AND A SUNSCREEN FOR THE CURE AND PREVENTION OF IRRITATION, INFLAMMATION AND CUTANEOUS ERYTHEMA**

KOSMETISCHE UND/ODER PHARMAZEUTISCHE ZUBEREITUNG ENTHALTEND DIMTHYLSULPHONE UND EINEN SONNENFILTER FÜR DIE BEHANDLUNG UND DIE PRÄVENTION VON REIZUNGEN, ENTZÜNDUNGEN UND HAUTERYTHEMA

COMPOSITION COSMETIQUE ET/OU PHARMACEUTIQUE COMPRENANT DU SULPHONE DE DIMETHYLE ET UN FILTRE SOLAIRE DESTINEE AU TRAITEMENT ET A LA PREVENTION D'IRRITATIONS, D'INFLAMMATIONS ET D'ERYTHEMES CUTANES

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(43) Date of publication of application:
**05.04.2006 Bulletin 2006/14**

(73) Proprietor: **De Paoli Ambrosi, Gianfranco**
**25010 San Felice del Benaco (BS) (IT)**

(72) Inventor: **De Paoli Ambrosi, Gianfranco**
**25010 San Felice del Benaco (BS) (IT)**

(74) Representative: **Long, Giorgio et al**
**Jacobacci & Partners S.p.A.**
**Via Senato, 8**
**20121 Milano (IT)**

(56) References cited:
EP-A- 1 230 911     WO-A-94/05272
WO-A-94/05293     WO-A-94/05301

## Description

### FIELD OF THE INVENTION

The present invention has as subject a new composition for cosmetic or pharmaceutical use intended for external use to be applied both to undamaged and damaged skin or onto the mucosa to reduce or inhibit irritation, inflammation and cutaneous erythema induced by events of an exogenous nature (irritations induced by chemical agents, pharmaceuticals, cosmetic ingredients, physical agents - such as, for example, solar radiation, ultraviolet radiation, ionising radiatiori, X rays, gamma rays, laser light - bacterial agents, viral agents, etc) and to photoprotect the skin from solar radiation and specifically from damage induced by ultraviolet radiation of types A, B and C.

### STATE OF THE ART

Erythema is a reddening of the skin caused by the increased delivery of blood to the blood vessels of the superficial dermis. Erythema is defined as active when it conveys the dilation of arterial vessels: showing a vivid red complexion and an increase in localised temperature can be detected. Passive erythema is due to venal stasis, showing a bluish-red complexion with a reduction in local temperature. Erythema has various dimensions, shapes and localisations; disappearing with vitropressure due to the interruption in blood flow. It can manifest itself for physical (mechanical, thermal, radiant), chemical, infective (mycosis, exanthematous infantile diseases), emotive (sudden reddening, localised to the face and neck occurring in embarrassing situations) causes, or be provoked by drugs, disvitaminosis, endocrine dysfunction or - even - appear over the course of an allergic reaction. The inflammation is a defensive response by the body towards tissue lesions by biological agents (microorganisms), chemical agents, physico-mechanical agents (for example trauma, radiation), or as a consequence of diseases. It has the biological function of rendering ineffective or destroying noxious factors of chemical, physical, biological natures, and successively to repair possible damage suffered by the effected tissues. The various vascular phenomena are determined by chemical mediators (mediators of inflammation) such as histamine, leukotrienes, prostaglandins, thromboxanes, interleukins.

Photo-protection consists in the putting into action of measures able to reduce the noxious effects of ultraviolet radiation on the skin.

### Inflammation and oxidative species

The Reactive Oxygen Species (ROS) are toxic and highly reactive molecules which play an important role in the genesis and the maintenance of inflammatory processes.

The superoxide radical $(O_2)$ is produced by monocytes and macrophages, cells involved in the inflammatory processes, and plays a role in the amplification of the process itself.

The hydroxide radical $(OH^{\bullet})$ and hydrogen peroxide radical $(H_2O_2^{\bullet})$ intervene in the inflammatory processes and are released during the metabolism of prostaglandins and in particular during the enzymatic metabolism of arachidonic acid.

The inflammatory processes are localised at the dermal level.

The problem on which the present invention is based is that of making available a cosmetic and/or pharmaceutical composition for the cure and/or prevention of inflammation, irritation and cutaneous erythema and to photoprotect the cuteous from solar radiation and in a more specific sense from the damage induced by ultraviolet radiation of types A, B and C.

Such a problem is solved by compositions containing dimethyl sulphone such as these delineated in the attached claims.

### BRIEF DESCRIPTION OF THE FIGURES

Fig. 1 shows the reflectance spectra of the skin obtained prior (curve a) and following (curve b) exposure to UVB radiation;

Fig. 2 shows the variations of the index of erythema ($\Delta$I.E.) as a function of time obtained from the non treated sites (control) and from the sites treated with the formulation SALDMS 5;

Fig. 3 shows the percentage of cutaneous erythema inhibition (P.I.E.) obtained with the formulation SALDMS 5 by the applicant and with the placebo.

DESCRIPTION OF THE INVENTION

The dimethyl sulphone, appropriately vehicularised for topical use, is used to prevent and/or cure cutaneous irritations induced by chemical, physical, bacterial and viral agents. The dimethyl sulphone is also used to inhibit erythema induced by physical agents such as, for non exhaustive indication, ultraviolet radiation (solar radiation), especially when associated with solar filters, ionising radiation, X rays, gamma rays, laser light (of whatever intensity and nature).

The dimethyl sulphone is also used to reduce the cutaneous irritation caused by chemical agents such as carboxylic acids, bicarboxylic acids, tricarboxylic acids, monocarboxylic alpha hydroxyacids, bicarboxylic alpha hydroxyacids, tricarboxylic alpha hydroxyacids, monocarboxylic beta hydroxyacids, bicarboxylic beta hydroxyacids, tricarboxylic beta hydroxyacids, resorcine, phenol, retinoic acid, adapalene, azelaic acid, salicylic acid, trichloroacetic acid, benzyl peroxide and other substances which can be used in the cosmetic and/or pharmaceutical field and which are characterised as potential irritants.

The dimethyl sulphone is also used for a specific photoprotective action able to reduce the damage induced by solar radiation, and ultraviolet radiation of types A, B and C, both of natural and artificial origins.

The invention refers in particular to a composition for the aforementioned use which is characterised by comprising dimethyl sulphone, used in a percentage by weight of from 0.5% to 90%, preferably between 5 and 60% w/w, still more preferably between 8% and 30% by weight. The percentage of dimethyl sulphone used will generally depend on the typology of application and on the fact of whether the intended use is for the prevention or the cure of the aforecited cutaneous manifestations.

Within the scope of the present invention are comprised compositions in which the dimethyl sulphone is used in combination with solar filters or compounds for favouring sun tanning or pharmaceutical substances whose irritation potential should be counteracted.

When the dimethyl sulphone is in combination with a solar filter (for example, PABA, Homosalate, Camphor, benzalkonium, methosulphate, benzophenone-3, phenylbenzimidazole sulphonic acid, Butyl methoxydibenzoylmethane, terephthalylidene dicamphor sulphonic acid, benzylidene camphor sulphonic acid, octocrylene, octyl methoxycinnamate, polyacrylamidomethyl benzylidene, PEG-25 PABA, octyl salicylate, Octyl dimethyl PABA, isoamyl p-methoxycinnamate, benzophenones-4, 3-Benzylidene camphor, 4-methylbenzylidene camphor, isopropylbenzyl salicylate, Octyltriazonesia), the quantity of dimethyl sulphone is comprised of between 0.5 % and 50 % by weight and the quantities of solar filter are comprised of between 1 % e 20 % and in any case within the limits for use imposed by the various regulations at the international level.

Wen the dimethyl sulphone is in combination with a pharmaceutically active ingredient endowed with an irritant activity towards the cuteous (for example retinoic acid, salicylic acid, azelaic acid, adapalene, benzoyl peroxide, metronidazole, antibiotics, sulphamidics, including their respective salts and esters, the dextrorotatory and/or levorotatory forms, racemic mixtures and -cis or -trans forms), the quantity of dimethyl sulphone is comprised of between 1.0 % and 60.0 % by weight and the quantity of irritant is comprised of between 5.0 % and 70 % by weight.

Within the compositions of the invention the weight balance up to 100% will be obtained through the addition of solvents such as water (in particular demineralised water), alcohols (such as ethyl alcohol) or glycols (for example, ethylene glycol or propylene glycol) - and/or excipients such as emulsifiers, antioxidants, lipid based excipients (fluid lipids or solid lipids), consistency factors, sequestering substances, preservatives. Such excipients, used in particular for the preparation of emulsions, gels, creams, ointments, etc., are widely known to the expert in the field and will therefore not be described in further detail.

EXPERIMENTAL SECTION

In corroboration of the present invention, experiments relating to the evaluation of the photoprotective action and to the ultraviolet radiation induced erythema inhibiting effect are reported.

UVB radiation induced cutaneous erythema is considered to be a good model for evaluating the damage produced to the skin by both chronic and acute exposure to solar radiation.

In this experiment, using a protocol already reported in the literature, the capacity of a formulation based on 5% dimethyl sulphone (SALDMS 5) to inhibit the cutaneous erythema induced, in healthy volunteers, by exposure to UVB radiation has been determined. For a more objective and quantitative evaluation, the erythematogenic course has been monitored using a non invasive technique such as reflectance spectrophotometry.

Experimental protocol

For the evaluation of the photoprotective and antierythema capacity of the formulation based on 5% dimethyl sulphone, twelve healthy volunteers, previously informed about the nature of the experiment and of the procedures employed, have been used. The volunteers, from whom written consent has been requested, have been selected from subjects having phototypes II and III.

Cutaneous erythema has been induced using a Mod. UVM-57 (UVP, San Gabriel, CA) ultraviolet lamp, capable of emitting radiation within the interval of 290-320 nm with a peak at 302 nm. For each subject, the minimum erythematogenic dose (MED) has been preliminarily determined and therefore, on the central section of each forearm, have been identified and demarcated, six cutaneous sites of 1 cm$^2$, which have been irradiated to provoke erythema, with exposure times equal to twice the MED corresponding to each subject.

Following the UVB irradiation, two sites have been used as controls and therefore not treated and the remaining sites have been treated in double with 100 mg of the formulation under test (SALDMS 5). The formulation has been applied to the cutaneous sites using appropriate chambers (Hill Top Chambers - Hill Top, Cincinnati, OH) for a period of three hours.

On completion of the period of treatment (three hours), each site, following removal of the Hill Top Chambers, has been washed with water to eliminate the residues of the formulation and left to rest for 30 minutes. For each site the erythema has been monitored over the successive 60 hours with an X-Rite mod.968 reflectance spectrophotometer. The instrument has been calibrated according to a white standard conforming to that envisaged by the National Bureau of Standards using a type C illumination source and an angle of observation of 2°. The spectrophotometer was connected to a personal computer which, using software supplied with the instrument (Spectrostart), was able to elaborate reflectance spectra of the skin in the 400-700 nm region.

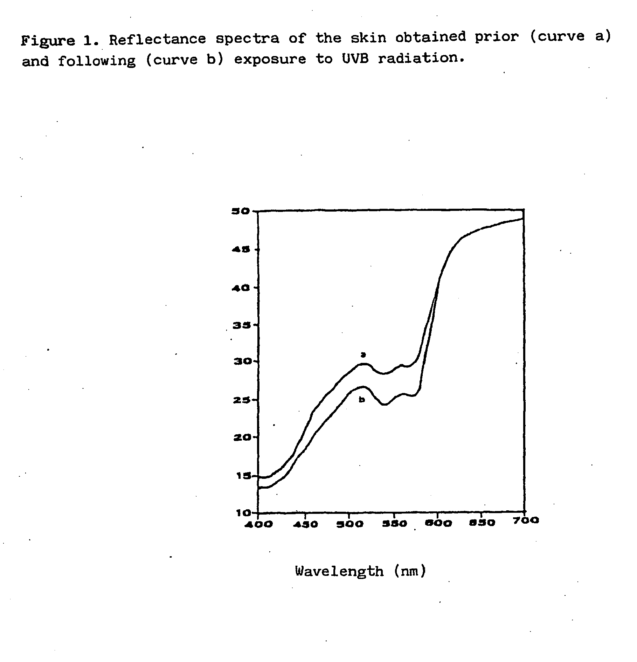

In Fig. 1 are reported the reflectance spectra relating to the same cutaneous site before (curve a) and after (curve b) exposure to the UVB radiation. As can be ascertained from curve b, the reflectance spectrum of the cutaneous site following exposure to the UVB radiation shows two bands of absorbance: a singlet at approx. 400 nm and another doublet between 540 and 580 nm, related to the absorbance of haemoglobin.

From the spectral data supplied by the instrument, it has been possible to calculate over time, for each cutaneous site tested and using the following equation:

$$I.E. = 100\left[\log\frac{1}{R560} + 1,5\left(\log\frac{1}{R540} + \log\frac{1}{R580}\right) - 2\left(\log\frac{1}{R510} + \log\frac{1}{R610}\right)\right]$$

the value of the erythema index (I.E.) which represents an important parameter proposed by Dawson for quantitatively monitoring cutaneous erythema.

In the equation set out above are summed, the values of the logarithms of the reciprocals of the reflectances of these wavelengths (540 nm, 560 nm, 580 nm) at which haemoglobin absorbance peaks are verified, whilst the corresponding values of the wavelengths (510 nm and 610 nm), the absorbance at which is principally due to the presence of melanin, are subtracted.

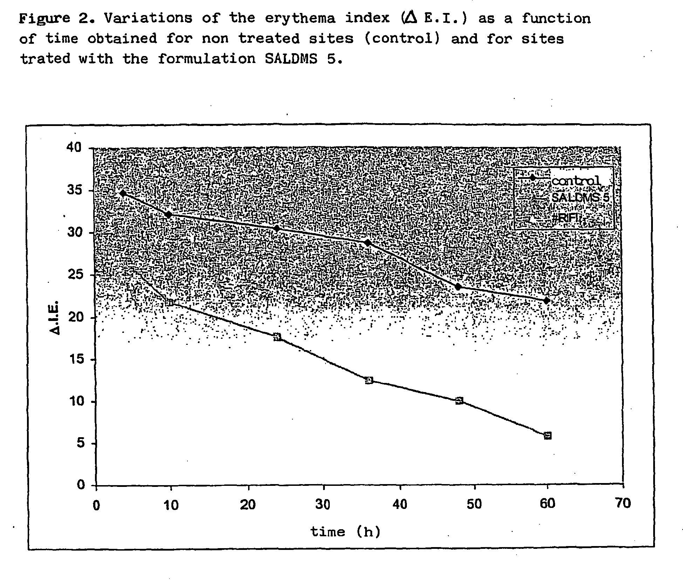

The E.I. baseline values, determined for each site prior to exposure to UVB radiation have been subtracted from the E.I. values, calculated at the different times for the same site, in such a manner obtaining the typical curves (ΔE.I. - time, see Fig. 2) from which have been calculated the corresponding areas under the curves (AUC). The AUC values hold particular importance in the evaluation of erythema in as much as they are inversely proportional to intensity and the duration of the erythema itself and therefore to the capacity of the product to inhibit erythema formation.

Hence, in order to better compare the efficacy of the individual formulations, the percentage inhibition of erythema (P.I.E.) has been calculated using the formula reported below.

$$P.I.E.\% = \frac{AUC_{(C)} - AUC_{(T)}}{AUC_{(C)}} \times 100$$

The AUC values represent the areas under the ΔE.I. - time curves of the treated sites [AUC (T)] or the control sites [AUC (C) ]. Statistical analyses of the results have been carried out using the Student t-test method.

## Results

Fig. 2 shows some typical curves, relating to subject 1, obtained by reporting the variations of the erythema index as a function of time both for the non treated sites (control) and for these treated with the SALDMS 5 formulation.

From the variation in the erythematogenic course, reported in Fig. 2, the inhibitory effect exercised, at different degrees, by the formulations tested in comparison to the erythema induced by UV B irradiation, is quite evident.

In table 1, are reported the mean AUC values obtained from the ΔE.I. - time curves for the dimethyl sulphone based formulations and for the individual subjects.

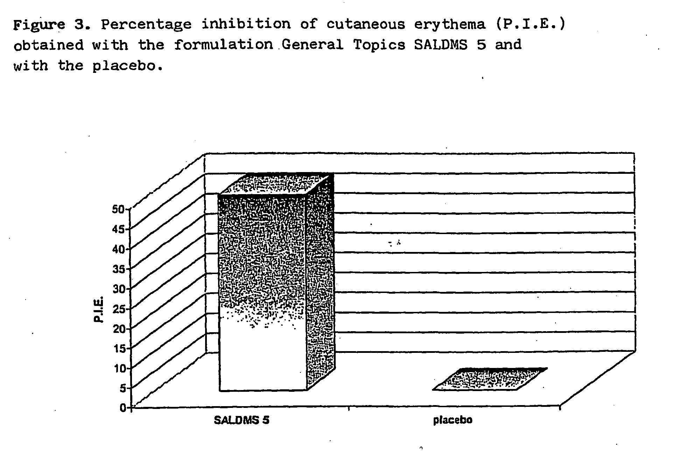

From the results obtained it is evident that the SALDMS 5 formulation has notable efficacy in the inhibition of cutaneous erythema induced by UVB radiation. To quantify the erythema inhibition capacity for the evaluated products, the values of the percentage inhibition of erythema (P.I.E.) have been calculated which are reported in figure 3.

In conclusion, from the results obtained in this experiment, the SALDMS 5 formulation has an interesting photoprotective effect, useful in the protection of the skin from the degenerative effects provoked by the actions of ultraviolet radiation.

The high antierythema and photoprotective capacity of the SALDMS 5 formulation allows for the efficacious use of this active ingredient in the cosmetic and pharmaceutical fields and in particular in the prevention and protection from the damage induced by ultraviolet radiation.

Tab. 1 - AUC values obtained for the control sites (non treated) and for the sites treated with the 5% dimethyl sulphone based formulation.

| Subject | Control | SALDMS 5 |
|---|---|---|
| A | 1580.2 | 828.4 |
| B | 1468.5 | 726.3 |
| C | 1698.3 | 864.1 |
| D | 1152.4 | 952.7 |
| E | 1338.2 | 764.5 |
| F | 1637.5 | 686.7 |
| G | 1212.5 | 921.54 |
| H | 1692.3 | 623.7 |
| I | 1547.8 | 721.6 |
| L | 1463.0 | 864.9 |
| M | 1865.5 | 786.3 |
| N | 1545.3 | 505.1 |
| Mean | 1516.8 | 770.5 |
| S.D. | 206.3 | 128.2 |
| P.I.E | ------ | 49.2 |

\* Percentage inhibition of erythema
$p < 0.01$ : SALDMS 5 versus controls

EXAMPLES OF FORMULATIONS

Preparation 1 - lotion/solution

| N° | Description | % w/w a |
|---|---|---|
| 01 | Dimethyl isosorbide | 40.00 |
| 02 | Pyruvic acid | 50.00 |
| 03 | Dimethyl sulphone | 10.00 |

Method of preparation: dissolve 03 in 01, to the solution obtained, mix in 02

Preparation 2 - lotion/solution

| N° | Description | % w/w a |
|---|---|---|
| 01 | Dimethyl isosorbide | 40.00 |
| 02 | Trichloroacetic acid | 50.00 |

(continued)

| N° | Description | % w/w |
|---|---|---|
| 03 | Dimethyl sulphone | 10.00 |

Method of preparation: dissolve 03 in 01, to the solution obtained, mix in 02

Preparation 3 - lotion/solution

| N° | Description | % w/w a |
|---|---|---|
| 01 | Dimethyl isosorbide | 20.00 |
| 02 | Glycolic acid | 50.00 |
| 03 | Dimethyl sulphone | 20.00 |
| 04 | Water | 10.00 |

Method of preparation: dissolve 03 + 02 in 01; mix the solution obtained with 04

Preparation 4 - oil in water emulsion

| N° | Description | % w/w a |
|---|---|---|
| | PHASE A | |
| 01 | Sreareth 2 | 3.00 |
| 02 | Steareth 21 | 2.00 |
| 03 | Ppg 15 stearyl ether | 10.00 |
| 04 | Tocopheryl acetate | 1.00 |
| 05 | Jojoba oil | 2.00 |
| 06 | Bht | 0.01 |
| 07 | Ascorbyl palmitate | 0.10 |
| 08 | Octyl methoxycinnamate | 5.00 |
| 09 | 4-methylbenzylidene camphor | 2.00 |
| | PHASE B | |
| 10 | Propylene glycol | 2.00 |
| 11 | Retinoic acid | 0.025 |
| 12 | Demineralised water | 10.00 |
| | PHASE C | |
| 13 | Dimethyl sulphone | 10.00 |
| 14 | Propylene glycol | 2.00 |
| 15 | Disodium EDTA | 0.07 |
| 16 | Glycerol | 5.00 |
| 17 | Phenoxyethanol | 1.00 |
| 18 | Methyl paraben | 0.10 |
| 19 | Ethyl paraben | 0.10 |
| 20 | Propyl paraben | 0.10 |
| 21 | Demineralised water qba | 100 |

Method of preparation: heat PHASE A) to 75°C; heat PHASE C) to +75°C; combine PHASE A) with PHASE C)with stirring to make a homogeneous solution; cool to +45°C; then add PHASE B) still with continual stirring and cool to 25°C.-

Preparation 5 - oil in water emulsion

| N° | Description | % w/w a |
|----|-------------|---------|
| | PHASE A | |
| 01 | Sreareth 2 | 3.00 |
| 02 | Steareth 21 | 2.00 |
| 03 | Ppg 15 stearyl ether | 10.00 |
| 04 | Tocopheryl acetate | 1.00 |
| 05 | Jojoba oil | 2.00 |
| 06 | Bht | 0.01 |
| 07 | Ascorbyl palmitate | 0.10 |
| 08 | Ethyl lactate | 5.00 |
| | PHASE B | |
| 09 | Propylene glycol | 2.00 |
| 10 | Azelaic acid | 15.0 |
| 11 | Demineralised water | 10.00 |
| | PHASE C | |
| 12 | Dimethyl sulphone | 10.00 |
| 13 | Propylene glycol | 2.00 |
| 14 | Disodium EDTA | 0.07 |
| 15 | Glycerol | 5.00 |
| 16 | Phenoxyethanol | 1.00 |
| 17 | Methyl paraben | 0.10 |
| 18 | Ethyl paraben | 0.10 |
| 19 | Propyl paraben | 0.10 |
| 20 | Demineralised water | 100 qba |

Method of preparation: heat PHASE A) to 75°C; heat PHASE C) to +75°C; combine PHASE A) with PHASE C) with constant stirring homogenising the solution; cool to +45°C; then combine with PHASE B) still with constant stirring and cooling to 25°C.

Preparation 6 - oil in water emulsion

| N° | Description | % w/w |
|----|-------------|-------|
| | PHASE A | a |
| 01 | Sreareth 2 | 3.00 |
| 02 | Steareth 21 | 2.00 |
| 03 | Ppg 15 stearyl ether | 10.00 |
| 04 | Tocopheryl acetate | 1.00 |
| 05 | Jojoba oil | 2.00 |
| 06 | Bht | 0.01 |
| 07 | Ascorbyl palmitate | 0.10 |
| 08 | Ethyl pyruvate | 5.00 |
| | PHASE B | |

(continued)

| N° | Description | % w/w a |
|---|---|---|
| | PHASE A | |
| 09 | Propylene glycol | 2.00 |
| 10 | Benzoyl peroxide | 5.00 |
| 11 | Demineralised water | 10.00 |
| | PHASE C | |
| 12 | Dimethyl sulphone | 10.00 |
| 13 | Propylene glycol | 2.00 |
| 14 | Disodium EDTA | 0.07 |
| 15 | Glycerol | 5.00 |
| 16 | Phenoxyethanol | 1.00 |
| 17 | Methyl paraben | 0.10 |
| 18 | Ethyl paraben | 0.10 |
| 19 | Propyl paraben | 0.10 |
| 20 | Demineralised water qba | 100 |

Method of preparation: heat PHASE A) to 75°C; heat PHASE C) to +75°C; combine PHASE A) with PHASE. C) with constant stirring homogenising the solution; cool to +45°C; then combine with PHASE B) still with constant stirring and cooling to 25°C.

Preparation 7 - oil in water emulsion

| N° | Description | % w/w a |
|---|---|---|
| | PHASE A | |
| 01 | Sreareth 2 | 3.00 |
| 02 | Steareth 21 | 2.00 |
| 03 | Ppg 15 stearyl ether | 10.00 |
| 04 | Tocopheryl acetate | 1.00 |
| 05 | Jojoba oil | 2.00 |
| 06 | Bht | 0.01 |
| 07 | Ascorbyl palmitate | 0.10 |
| 08 | Retinoic acid | 0.02 |
| | PHASE B | |
| 09 | Propylene glycol | 2.00 |
| 10 | Lactic acid | 10.00 |
| 11 | Demineralised water | 10.00 |
| | PHASE C | |
| 12 | Dimethyl sulphone | 10.00 |
| 13 | Propylene glycol | 2.00 |
| 14 | Disodium EDTA | 0.07 |
| 15 | Glycerol | 5.00 |
| 16 | Phenoxyethanol | 1.00 |
| 17 | Methyl paraben | 0.10 |
| 18 | Ethyl paraben | 0.10 |
| 19 | Propyl paraben | 0.10 |

(continued)

| N° | Description | % w/w a |
|---|---|---|
| | PHASE C | |
| 20 | Demineralised water qba | 100 |

Method of preparation: heat PHASE A) to 75°C; heat PHASE C) to +75°C; combine PHASE A) with PHASE C) with constant stirring homogenising the solution; cool to +45°C; then combine with PHASE B) still with constant stirring and cooling to 25°C.

Preparation 8 - oil in water emulsion

| N° | Description | % w/w a |
|---|---|---|
| | PHASE A | |
| 01 | Sreareth 2 | 3.00 |
| 02 | Steareth 21 | 2.00 |
| 03 | Ppg 15 stearyl ether | 10.00 |
| 04 | Tocopheryl acetate | 1.00 |
| 05 | Jojoba oil | 2.00 |
| 06 | Bht | 0.01 |
| 07 | Ascorbyl palmitate | 0.10 |
| 08 | Ethyl pyruvate | 5.00 |
| | PHASE B | |
| 09 | Propylene glycol | 2.00 |
| 10 | Adapalene | 0.20 |
| 11 | Demineralised water | 10.00 |
| | PHASE C | |
| 12 | Dimethyl sulphone | 10.00 |
| 13 | Propylene glycol | 2.00 |
| 14 | Disodium EDTA | 0.07 |
| 15 | Glycerol | 5.00 |
| 16 | Phenoxyethanol | 1.00 |
| 17 | Methyl paraben | 0.10 |
| 18 | Ethyl paraben | 0.10 |
| 19 | Propyl paraben | 0.10 |
| 20 | Demineralised water qba | 100 |

Method of preparation: heat PHASE A) to 75°C; heat PHASE C) to +75°C; combine PHASE A) with PHASE C) with constant stirring homogenising the solution; cool to +45°C; the combine with PHASE B) still with constant stirring and cooling to 25°C.

Preparation 9 - oil in water emulsion

| N° | Description | % w/w a |
|---|---|---|
| | PHASE A | |
| 01 | Sreareth 2 | 3.00 |
| 02 | Steareth 21 | 2.00 |
| 03 | Ppg 15 stearyl ether | 10.00 |
| 04 | Tocopheryl acetate | 1.00 |

(continued)

| N° | Description | % w/w a |
|---|---|---|
| | PHASE A | |
| 05 | Jojoba oil | 2.00 |
| 06 | Bht | 0.01 |
| 07 | Ascorbyl palmitate | 0.10 |
| 08 | Ethyl pyruvate | 5.00 |
| | PHASE B | |
| 09 | Propylene glycol | 2.00 |
| 10 | Lactic acid | 10.00 |
| 11 | Demineralised water | 10.00 |
| | PHASE C | |
| 12 | Dimethyl sulphone | 10.00 |
| 13 | Propylene glycol | 2.00 |
| 14 | Disodium EDTA | 0.07 |
| 15 | Glycerol | 5.00 |
| 16 | Phenoxyethanol | 1.00 |
| 17 | Methyl paraben | 0.10 |
| 18 | Ethyl paraben | 0.10 |
| 19 | Propyl paraben | 0.10 |
| 20 | Demineralised water qba | 100 |

Method of preparation: heat PHASE A) to 75°C; heat PHASE C) to +75°C; combine PHASE A) with PHASE C) with constant stirring homogenising the solution; cool to +45°C; then combine with PHASE B) still with constant stirring and cooling to 25°C.

Preparation 10 - oil in water emulsion

| N° | Description | % w/w a |
|---|---|---|
| | PHASE A | |
| 01 | Steareth 2 | 3.00 |
| 02 | Steareth 21 | 2.00 |
| 03 | Ppg 15 stearyl ether | 10.00 |
| 04 | Tocopheryl acetate | 1.00 |
| 05 | Jojoba oil | 2.00 |
| 06 | Bht | 0.01 |
| 07 | Ascorbyl palmitate | 0.10 |
| 08 | Ethyl pyruvate | 5.00 |
| | PHASE B | |
| 09 | Propylene glycol | 2.00 |
| 10 | Lactic acid | 10.00 |
| 11 | Demineralised water | 10.00 |
| | PHASE C | |
| 12 | Dimethyl sulphone | 10.00 |
| 13 | Metronidazole | 2.00 |
| 14 | Disodium EDTA | 0.07 |
| 15 | Glycerol | 5.00 |

(continued)

| | PHASE C | |
|---|---|---|
| 16 | Phenoxyethanol | 1.00 |
| 17 | Methyl paraben | 0.10 |
| 18 | Ethyl paraben | 0.10 |
| 19 | Propyl paraben | 0.10 |
| 20 | Demineralised water qba | 100 |

Method of preparation: heat PHASE A) to 75°C; heat PHASE C) to +75°C; combine PHASE A) with PHASE C) with constant stirring homogenising the solution; cool to +45°C; then combine with PHASE B) still with constant stirring and cooling to 25°C.

**FIGURES**

Figure 1.

Reflectance spectra of the skin obtained prior (curve a) and following (curve b) exposure to UVB radiation. X axis - Wavelength (nm)

Figure 2.

Variations of the erythema index ($\Delta$E.I.) as a function of time obtained for non treated sites (control) and for sites treated with the formulation SALDMS 5.
Y axis - $\Delta$E.I.
X axis - time (hours)
Legend - Control, SALDMS 5, #RIFI

Figure 3.

Percentage inhibition of cutaneous erythema (P.I.E.) obtained with the formulation General Topics SALDMS 5 and with the placebo.
Y axis - P.I.E.
X axis - SALDMS 5, Placebo

**Claims**

1. A composition containing a solar filter and dimethyl sulphone, wherein the quantity of dimethyl sulphone is comprised of between 0,5 % and 50 % by weight and the quantities of solar filters are comprised of between 1 % and 20 % by weight.

2. The composition according to claim 1, wherein said solar filter is selected from PABA, Horaosalate, Camphor, benzalkonium, methosulphate, benzofenone-3, Phenylbenzimidazole sulphonic acid, Butyl methoxydibenzoylmethane, Terephthalylidene dicamphor sulphonic acid, Benzylidene camphor sulphonic acid, Octocrylene, Octyl methoxycinnamate, Polyacrylamidomethyl benzylidene, PEG-25 PABA, Octyl salicylate, Octyl dimethyl PABA, Isoamyl p-methoxycinnamate, Benzophenone-4, 3-Benzylidene camphor, 4-methylbenzylidene camphor, isopropylbenzyl salicylate, Octyl-triazonesia,

**Patentansprüche**

1. Eine Zusammensetzung, die ein Solarfilter und Dimethylsulfon enthält, wobei die Menge an Dimethylsulfon zwischen 0,5 und 50 Gewichtsprozent liegt und die Mengen an Solarfiltern zwischen 1 und 20 Gewichtsprozent liegen.

2. Die Zusammensetzung gemäß Anspruch 1, bei der das Solarfilter aus PABA, Homosalat, Kampfer, Benzalkonium, Methosulfat, Benzofenon-3, Phenylbenzimidazolsulfonsäure, Butylmethoxydibenzoylmethan, Terephthalyliden-Dikampfer-Sulfonsäure, Benzylidenkampfersulfonsäure, Octocrylen, Octylmethoxycinnamat, Polyacrylamidomethyl-Benzyliden, PEG-25 PABA, Octylsalicylat, Octyldimethyl-PABA, Isoamyl-p-methoxycinnamat, Benzophenon-4, 3-Benzylidenkampfer, 4-Methylbenzylidenkampfer, Isopropylbenzylsalicylat, Octyl-Triazonesia ausgewählt ist.

**Revendications**

1. Composition contenant un filtre solaire et de la diméthylsulfone, dans laquelle la quantité de diméthylsulfone est comprise dans l'intervalle de 0,5 % à 50 % en poids et les quantités de filtres solaires sont comprises dans l'intervalle de 1 % à 20 % en poids.

2. Composition suivant la revendication 1, dans laquelle ledit filtre solaire est choisi entre le PABA, l'homosalate, le camphre, le méthosulfate de benzalkonium, la benzofénone-3, l'acide phénylbenzimidazolesulfonique, le butylméthoxydibenzoylméthane, l'acide téréphtalylidène-dicamphosulfonique, l'acide benzylidènecamphosulfonique, l'octocrylène, le méthoxycinnamate d'octyle, le poly-acrylamidométhylbenzylidène, le PEG-25-PABA, le salicylate d'octyle, l'ocyldiméthylPABA, le p-méthoxycinnamate d'isoamyle, la benzophénone-4, le 3-benzylidène-camphre, le 4-méthylbenzylidène-camphre, le salicylate d'isopropylbenzyle et l'octyl-triazonésie.

Figure 1. Reflectance spectra of the skin obtained prior (curve a) and following (curve b) exposure to UVB radiation.

Wavelength (nm)

Figure 2. Variations of the erythema index ($\Delta$ E.I.) as a function of time obtained for non treated sites (control) and for sites trated with the formulation SALDMS 5.

Figure 3. Percentage inhibition of cutaneous erythema (P.I.E.) obtained with the formulation General Topics SALDMS 5 and with the placebo.